# EUROPEAN PATENT SPECIFICATION

(11) **EP 3 980 106 B1**
(45) Date of publication and mention of the grant of the patent: **19.03.2025**
(21) Application number: 19931864.3
(22) Date of filing: 06.06.2019
(51) Int. Cl.: A61M 16/16, A62B 7/00, A61M 16/00, A61M 16/10, G01N 25/66

(54) **DEW POINT SENSOR SYSTEM, METHOD FOR MODULATING A HUMIDIFIER HEATER PLATE, AND HUMIDIFIER/MEDICAL DEVICE CONTAINING THE SAME**
TAUPUNKTSENSORSYSTEM, VERFAHREN ZUR MODULATION EINER BEFEUCHTERHEIZPLATTE UND DAZUGEHÖRIGE(R) BEFEUCHTER/MEDIZINISCHE VORRICHTUNG
SYSTÈME DE CAPTEUR DE POINT DE ROSÉE, PROCÉDÉ DE MODULATION D'UNE PLAQUE CHAUFFANTE D'HUMIDIFICATEUR, ET HUMIDIFICATEUR/DISPOSITIF MÉDICAL LE CONTENANT

(43) Date of publication of application: 13.04.2022
(73) Proprietor: Vincent Medical (Dong Guan) Manufacturing Co., Ltd., Dongguan, Guangdong 523730 (CN); Vincent Medical (Dong Guan) Technology Co., Ltd, Dongguan, Guangdong 523808 (CN)
(72) Inventor: XU, Jiebing, Dongguan, Guangdong 523730 (CN); YU, Haibin, Dongguan, Guangdong 523730 (CN); ZHAO, Jun, Dongguan, Guangdong 523657 (CN); ZHU, Wei, Shenzhen, Guangdong 518000 (CN)
(74) Representative: Prinz & Partner mbB
(86) International application number: PCT/CN2019/090385
(87) International publication number: WO 2020/243954

(56) References cited:
- EP-A1- 3 542 868
- CN-A- 102 105 189
- CN-A- 108 245 759
- SU-A1- 1 442 895
- US-A- 5 558 084
- US-A1- 2008 308 100
- US-A1- 2011 120 462
- US-A1- 2013 081 621
- US-A1- 2013 263 851
- US-B2- 10 357 627

## Description

### TECHNICAL FIELD

The present invention relates to a dew point sensor system and a machine, such as a humidifier, containing such a dew point sensor system. More specifically, the present invention relates to a dew point sensor system containing an ambient temperature sensor.

### BACKGROUND

Humidifiers are used to provide humidified air to a user, typically patients in hospitals, hospices, and even at home. Humidifiers are often operated in a variety of conditions, ranging at temperatures of from about 10 °C to about 40 °C and above. It is well-known that the amount of water potentially held in the air is dependent on the temperature of the air, and thus there is a distinction between relative humidity and absolute humidity.

Accordingly, humidifiers which measure the ambient air temperature are known as seen in, for example, US Pat. No. 8,616,202 B2 to Fisher &Paykel Healthcare, Ltd., published on December 31, 2013, and US Pat. No. 10,058,663 B2 to Fisher &Paykel Healthcare, Ltd., published on August 28, 2018.

Such references typically include an ambient temperature sensor within the housing of the device and/or attached to the housing of the device, such as, for example, on the external surface of the air passageway. However, it has now been found that such ambient temperature sensor locations do not provide accurate temperature readings of the ambient temperature, which can then adversely-affect the calculations of the dew point and cause the humidifier to provide an inaccurate amount of heating.

US 2011/0120462 A1 discloses a breathing assistance system for delivering a stream of heated, humidified gases to a user, including a humidifier unit which holds and heats a volume of water, a temperature sensor which measures the temperature of the gases exiting the humidifier unit, an ambient temperature sensor which measures the temperature of gases before they enter the humidifier unit, a flow sensor which measures the flow rate of the gases stream, a controller which receives data from the temperature and flow sensors, and determines a control output in response. The control output is determined from a look-up table loaded in the controller, and the look-up table loaded in the controller can calculate the O2 fraction in the blended humidified air.

US 5 558 084 A discloses a humidifier having an ambient temperature sensor for sensing the temperature of the ambient air in the environment in which the humidifier is located. A difference temperature selector is provided to allow a user to select a difference temperature which is the difference in temperature between the sensed ambient temperature and a comfortable and efficient temperature for supply of the humidified gases provided by the humidifier to a patient or user.

US 2013/0263851 A1 provides systems for humidifying gas delivered to a patient, including a humidifier unit which includes a chamber that receives water from a water source, a heat source disposed within the chamber, a water flow sensor, an output temperature sensor, an output humidity sensor, and/or other sensors. A controller receives flow data relating to water being provided to the chamber and temperature data, and adjusts a flow of water being provided to the chamber based on the flow data and/or a heat output of the heat source based on the received data.

US 2008/0308100 A1 discloses a patient treatment system including a patient circuit, a pressurizing flow module disposed in the patient circuit, a humidifier disposed in the patient circuit, a monitor, sensors within the patient circuit, and a processor. The patient circuit delivers a flow of gas from a gas source to an airway of a patient. The pressurizing flow module elevates the pressure of the gas within the patient circuit. The humidifier elevates the humidity of the gas in the patient circuit to a circuit humidity level. The monitor monitors at least one parameter of the gas from the gas source. The sensors generate corresponding signals that can be processed to estimate a rate at which vapor is added to the gas in the patient circuit. The processor determines the humidity level of the gas in the patient circuit downstream from the humidifier based on the signals generated by the sensors and the at least one parameter of the gas in the gas source. The humidity level may be determined based on a look-up table, and the maximum humidity level may be determined based on a look-up table.

Accordingly, it is desirable to provide a humidifier having an ambient temperature sensor which more accurately measures the ambient temperature. In addition, it is desirable to provide a humidifier and/or a medical device with a more accurate ambient temperature sensor.

### SUMMARY

The invention is set out in the claims.

### BRIEF DESCRIPTION OF DRAWINGS

Fig. 1 shows a schematic view of an embodiment of the electronic components of the dew point sensor system;
Fig. 2 shows an embodiment of a humidifier of the present invention; and
Fig. 3 shows an embodiment of the invention herein containing a blower.

The figures herein are for illustrative purposes only and are not necessarily drawn to scale.

### DETAILED DESCRIPTION

Unless otherwise specifically provided, all measurements are made in metric units. Furthermore, all percentages, ratios, etc. herein are by weight, unless specifically indicated otherwise.

As used herein the term "operatively-connected" indicates that the item (s) is (are) connected in a manner which allows them to operate. This may involve, for example, wires, a transmitter/receiver pair, a pair of transceivers, etc. This phrase may also indicate that a physical structure connects the various indicated items.

As used herein, the term "user" indicates the user of the invention herein, and may be, for example, an owner, a patient, a car-giver, etc.

Unless otherwise specifically described all of the devices, items and/or parts herein may be made from industry-standard materials which are available from multiple suppliers worldwide.

In an embodiment of the present invention, a dew point sensor system contains a controller, an ambient temperature sensor, an air pathway, a heater plate. The controller contains a dew point calculator, and the ambient temperature sensor generates ambient temperature data. The ambient temperature sensor is operatively-connected to the controller. The air pathway contains a blower, a flow sensor within the air pathway, a liquid reservoir operatively-connected to the air pathway, and component which is selected from an air pathway temperature sensor, a breathing circuit heating element, and a combination hereof. The blower causes the air to move in the air pathway, and the flow sensor generates air flow data from the air moving in the air pathway. The heater plate is operatively-connected to heat the liquid reservoir. The heater plate also contains a heater plate temperature sensor which measures the heater plate temperature and generates heater plate temperature data. An external wire is operatively-connected to the controller and the component.

In an embodiment herein, the user sets the desired air flow and the controller adjusts the blower accordingly. In an embodiment herein, if the air flow data shows that the air flow is different; or at least 5%different; or at least 10%different, from the desired air flow set by the user, then the controller may sound; or sounds, an alarm. In an embodiment herein, the controller sounds an alarm only if the difference between the air flow in the air pathway and the desired air flow exists for a continuous period of time of about ≥ 30 seconds; or about ≥ 1 minute; or about ≥ 2 minutes.

The ambient temperature sensor is located on the external wire. The heater plate and the heater plate temperature sensor are both operatively-connected to the controller. The controller references a dew point temperature. The controller also receives the ambient temperature data, the air flow data, and the dew point temperature to calculate the target heater plate temperature. The blower is optionally operatively-connected to the controller. The controller may modulate the heater plate temperature by, for example, increasing the heater plate temperature, decreasing the heater plate temperature, or both.

The controller may reference the dew point temperature by, for example, referring to a look-up table, calculating the dew point temperature, etc. as desired.

Without intending to be limited by theory, it has surprisingly-been found that an ambient temperature sensor connected within the housing of a humidifier within the air pathway is typically reading an inaccurate temperature. Even when the ambient temperature sensor is located in, or adjacent-to, the air pathway before the liquid chamber/humidification chamber, it has been found that the temperature within the housing itself and/or the surface of the housing, and the air pathway connected thereto are often warmer than the ambient temperature because of the heat radiated by the blower, the heater plate, the internal electronics and power system, etc. and the temperature sensed may be higher, or even significantly-higher, for example, up to 10 °C higher, than the actual ambient temperature. Surprisingly, it has been found that even when the ambient temperature sensor is connected to the external surface of the air pathway, the temperature sensed is often higher than the actual ambient temperature.

Such an inaccurate ambient temperature data would of course, then adversely-affect the controller's dew point calculation, and therefore could cause, for example, increased condensation in the air pathway, etc.

Accordingly, in an embodiment herein, the ambient temperature sensor is affixed to the external wire at a position external to the housing, so as to sense the ambient temperature of the air which is being drawn into the housing and therefore the air pathway, by the blower. In an embodiment herein, the external wire does not conduct electricity to or from any heating elements within, external to, or adjacent to the air pathway. Without intending to be limited by theory it is believed that a wire which conducts electricity to or from a heating element, such as those within, external to, or adjacent to an air pathway, may also become warm and therefore throw off the ambient temperature data generated by the ambient temperature sensor.

In an embodiment herein, the dew point sensor system further contains a housing. The housing typically contains, or encompasses the air pathway, the heater plate, and the controller. The liquid reservoir may be partially-contained within the housing, affixable to the housing, etc. and typically the liquid reservoir directly-contacts the heater plate.

In an embodiment herein, the air pathway temperature sensor is located within the air pathway downstream of the liquid reservoir. Alternatively, the air pathway temperature sensor is located within the air pathway immediately downstream of the liquid reservoir. Alternatively, the air pathway temperature sensor is located downstream of the liquid reservoir and is proximate to; or immediately upstream of, the user.

In an embodiment herein, the controller employs; or contains; or references, a dew point calculator to calculate the dew point from the ambient temperature data, the air flow data, and the heater plate temperature data. In an embodiment herein, the dew point calculator is a look-up table to calculate the dew point temperature from the ambient temperature data, the air flow data, and the heater plate temperature data. Typically, the dew point temperature is calculated as a function of the air flow rate within the air pathway, the heater plate temperature, and the ambient (room) temperature. So, for a given flow rate set by the user (i.e., "desired air flow"), ambient temperature, and a given dew point temperature set by the user; the controller can calculate the target heater plate temperature and the controller then adjusts the power flowing to the heater plate to achieve this temperature. In an embodiment herein, some data; or all data, is real-time data. In an embodiment herein, the controller adjusts the power flowing to the heater plate if the difference between the heater plate temperature and the target heater plate temperature is about > 0.1 °C. In such an embodiment, if the difference between the heater plate temperature and the target heater plate temperature is about ≤ 0.1 °C, then the controller does not adjust the power flowing to the heater plate.

In an embodiment herein, it may be desirable for the dew point system herein to contain multiple; or a plurality of air pathway temperature sensors. In an embodiment herein, a liquid reservoir-side air pathway temperature sensor is located within the air pathway and downstream; or immediately downstream, of the liquid reservoir, and a patient-side air pathway temperature sensor is located within the air pathway downstream of the liquid reservoir and proximate to; or immediately upstream of, the user.

The dew point is also dependent upon the speed of the air flowing in the air pathway, and therefore the dew point system herein contains a blower to move air through the air pathway and an air flow sensor to generate air flow data from the air moving in the air pathway. In an embodiment herein, the desired air flow (rate) is set at a specific speed by the user and then the controller adjusts the blower accordingly, so that this is removed as a variable -in effect, this is a constant air flow value that is stabilized by the controller. In an embodiment herein, the blower has a blower operating speed and the controller may reference; or references, the air flow data to modulate the blower operating speed. The controller may modulate the blower operating speed by, for example, increasing the blower operating speed, decreasing the blower operating speed, or both.

In an embodiment herein, due to the importance of receiving accurate heater plate temperature data, the heater plate temperature sensor is directly-affixed to the heater plate. Without intending to be limited by theory, it is believed that such an arrangement allows for a direct measurement of the heater plate temperature, instead of, for example, an estimation of the heater plate temperature via a measurement of the electrical load being used to heat up the heater plate.

In addition to modulating the heater plate temperature with respect to the dew point, the controller may also modulate the hear plate temperature in response to when the heater plate temperature data indicates an unacceptably high temperature, such as above about 120 °C; or above about 150 °C, etc. Such data would indicate that the water reservoir has run dry and therefore the heater plate should be shut off so as to avoid damage to the heater plate, the liquid reservoir, etc.

The liquid reservoir herein is typically a transparent or translucent container which is made of heat-resistant materials such as high density plastic, glass, metal, and a combination thereof; or polypropylene, polycarbonate, aluminium, and a combination thereof; or polycarbonate, aluminium, and a combination thereof. The liquid reservoir contains the liquid; or water; or distilled water; or sterile water, which is evaporated to create the humidified air. The liquid reservoir is operatively-connected; or fluidly-connected, to the air pathway so as to allow the typically can be opened so as to allow the user (or another person) to add a liquid; or water; or distilled water; or sterile water, thereto.

In an embodiment herein, the dew point sensor system may further contain an; or a plurality of, air pathway humidity sensor located within the air pathway which generate air pathway humidity data and transmits such air pathway humidity data to the controller.

In an embodiment herein, a humidifier contains the dew point sensor system described herein. In an embodiment herein, the dew point sensor system herein is contained within, or as a part of a medical device.

The dew point sensor system, humidifier, and/or medical device herein is typically operatively-connected to a power system such as a power supply, a battery, a power grid, an AC power outlet, etc.

### METHOD

In an embodiment herein, a method for modulating a humidifier contains the steps of providing a humidifier containing a dew point sensor system, generating ambient temperature data, transmitting the ambient temperature data to the controller, generating air flow data, transmitting the air flow data to the controller, generating heater plate temperature data, transmitting the heater plate temperature data to the controller, referencing a dew point temperature, transmitting the dew point temperature to the controller, receiving the ambient temperature data, the air flow data, the heater plate temperature data and the dew point temperature at; or by, the controller, calculating a target heater temperature based on the ambient temperature data, the air flow data, and the dew point temperature, and comparing the target heater plate temperature with the heater plate temperature data.

The controller calculates the target heater temperature based on the ambient temperature data, the air flow data (set by user), and a set dew point temperature. If the heater plate temperature data shows that the heater plate is lower than the target heater plate temperature, then the controller adjusts the heater plate temperature to the target heater plate temperature based on the target heater plate temperature and the heater plate temperature data. In an embodiment herein, the controller adjusts the heater plate temperature if the difference between the heater plate temperature and the target heater plate temperature is about ≥ 0.1 °C; or about ≥2 °C; or about ≥ 4 °C. In an embodiment herein, if the controller finds that the heater plate temperature is higher than the target heater plate temperature, then the controller may adjust the heater plate temperature by decreasing the power flowing to the heater plate. If the controller finds that the heater plate temperature is lower than the target heater plate temperature, then the controller may adjust the heater plate temperature by increasing the power flowing to the heater plate. Alternatively, in either of the above cases, the controller may sound an alarm.

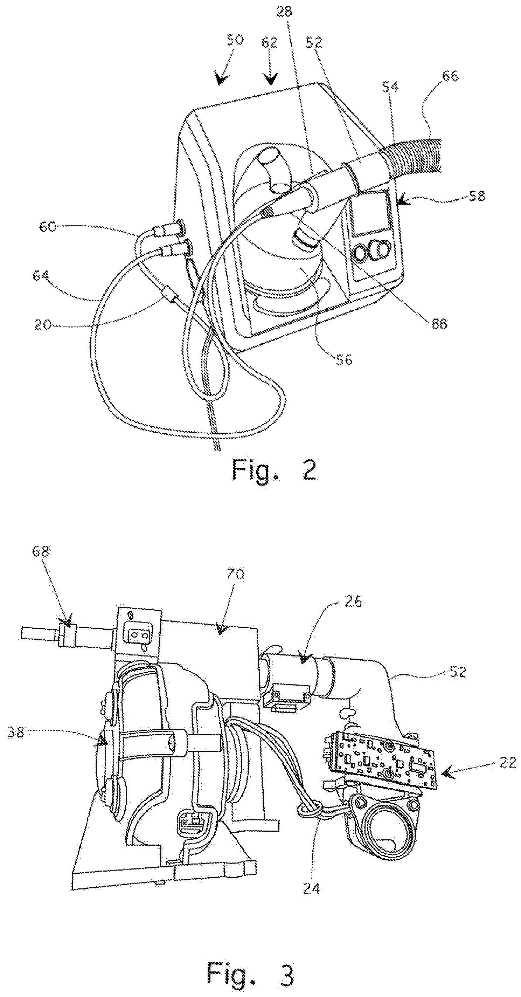

In an embodiment of the method herein, the dew point sensor system may further contain a breathing circuit (see Fig. 2 at 54), the breathing circuit comprises a heating circuit to heat the breathing gas. the dew point sensor system may further contain an air pathway temperature sensor which provides air pathway temperature data to the controller, and where the method may further include the step of the controller adjusting the power flowing to the heating circuit based upon the air pathway temperature data. The various types of data described herein (e.g., ambient temperature data, air flow data, heater plate temperature data, air pathway temperature data, air pathway humidity data, etc.) may be transmitted to the controller either via wires, or via, for example, radio or other wireless signals.

In an embodiment herein, the controller further references; or calculates, the real-time dew point temperature based on the heater plate temperature data, the air flow data, and the ambient temperature data. In an embodiment herein, if the controller discovers that the difference between the set dew point temperature data and the real-time dew point temperature is about ≥ 1 °C; or about ≥ 2 °C; or about ≥ 4 °C after warm-up (about 20 minutes after the heater plate is activated), then the controller sounds an alarm. In an embodiment of the invention herein, if the controller discovers that the difference between the set dew point temperature data and the real-time dew point temperature is about ≥ 1 °C; or about ≥ 2 °C; or about ≥ 4 °C after warm-up (about 20 minutes after the heater plate is activated) for a continuous period of time of about ≥ 30 seconds; or about ≥ 1 minute; or about ≥ 2 minutes, then the controller sounds an alarm.

In an embodiment herein, the alarm herein may be a notification, such as a sound, a light, a computer message, a wireless signal, and/or any other signal intended to notify and/or draw the attention of the user.

In an embodiment herein, the process further contains the step of repeating the steps from the generating of the ambient temperature data to the step of the controller adjusting the heater plate temperature to the target temperature based on the target temperature and the heater plate temperature data.

In an embodiment herein, the dew point sensor system herein generates ambient temperature data and sends this data to the controller. The blower starts up and air flow data is generated and transmitted to the controller. In an embodiment herein, the user sets a desired air flow and the controller adjusts the blower speed to provide the desired air flow. In an embodiment herein, if the air flow data shows that the air flow is different; or at least 5%different; or at least 10%different, from the desired air flow set by the user, then the controller may sound an alarm. The heater plate is activated by the controller and the heater plate temperature sensor generates heater plate temperature data which is transmitted to the controller. The controller references; or calculates, the target heater plate temperature based on the ambient temperature data, the air flow data and the set dew point temperature.

The controller then compares the heater plate temperature data with the target heater plate temperature. If the heater plate temperature data indicates the difference between the heater plate temperature and the target heater plate temperature is about ≥ 0.1 °C; or about ≥ 2 °C lower; or about ≥ 4 °C, then the controller adjusts the heater plate temperature by appropriately increasing or decreasing the heater plate temperature; or appropriately increasing or decreasing the power flowing to the heater plate.

In an embodiment of the method herein, the controller checks the system at a rate of at least about 600 times/minute; or from about 1 times/minute to about 1000 times/minute; or from about 10 times/minute to about 800 times/minute.

In an embodiment herein, after turning on the humidifier and/or medical device, the heater plate warms up for a predetermined period of time; or from about 1 minute to 35 minutes; or from about 2 minutes to about 30 minutes. The heater plate temperature data is generated in step (F) when the heater plate is activated.

In an embodiment herein, the medical device is selected from the group of a humidifier, a continuous positive air pressure machine, an automatic positive air pressure machine, and a combination thereof; or a humidifier.

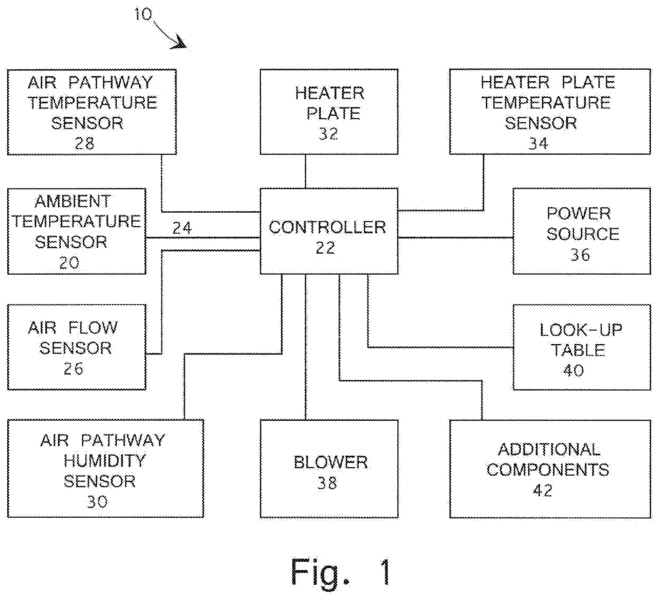

Turning to the figures, Fig. 1 shows a schematic view of an embodiment of the electronic components of the dew point sensor system, 10. An ambient temperature sensor, 20, is operatively-connected to a controller, 22, by a wire, 24. The ambient temperature sensor generates ambient temperature data and transmits it to the controller via the wire. An air flow sensor, 26, is also operatively-connected to the controller, 22, generates air flow data, and transmits it to the controller, 22. An air pathway temperature sensor, 28, is operatively-connected to the controller, 22, generates air pathway temperature data, and transmits it to the controller, 22. Also in Fig. 1, an air pathway humidity sensor, 30, is operatively-connected to the controller, 22, generates air pathway humidity data, and transmits it to the controller, 22.

A heater plate, 32, is operatively-connected to the controller, 22, as is a heater plate temperature sensor, 34. The controller, 22, modulates the heater plate temperature by adjusting the amount of electricity powering the heater plate, 32. The controller, 22, is also operatively- connected to a power source, 36, which delivers electrical power to the controller, 22. A blower, 38, is operatively-connected to the controller, 22. The controller, 22, modulates the blower operational speed by, for example, adjusting the amount of electricity provided to the blower, 38.

The controller, 22, is also operatively-connected to a look-up table, 40, which is used by the controller, 22, to calculate the target heater plate temperature and the dew point temperature. The controller, 22, may also be connected to one or more additional components, 42, which may provide additional functionality, such as a clock, an alarm, a water level sensor, a heating circuit for the breathing circuit, and a combination thereof.

Fig. 2 shows an embodiment of a humidifier, 50, of the present invention. An air pathway, 52, contains a breathing circuit, 54, a liquid reservoir, 56, a control panel, 58, and an external wire, 60, which is outside of a housing, 62, which contains, for example, the controller (see Fig. 1 at 22), the heater plate (see Fig. 1 at 32), the heater plate temperature sensor (see Fig. 1 at 34), the blower (see Fig. 1 at 36), etc. The ambient temperature sensor, 20, is located; i.e., attached to, the external wire, 60.

The embodiment of Fig. 2 also shows an optional heating circuit wire, 64, which provides electricity to a heating circuit, 66, located within the breathing circuit, 54. The external wire, 60, is connected to the air pathway temperature sensor, 28, which monitors the temperature at the humidifier-side of the breathing circuit, 54. The air pathway temperature sensor, 28, could also be located at the patient-end of the breathing circuit, 54.

Fig. 3 shows a side view of an embodiment of the blower, 38, herein. Fig. 2 shows the blower connected to part of the air pathway, 52, which contains the air flow sensor, 26, therein. Fig. 3 also shows an oxygen inlet, 68, upstream of a mixing chamber, 70, which combines and homogenizes air from the blower, 38, and oxygen from the oxygen inlet, 68. The controller, 22, is operatively-connected to the blower, by the wires, 24.

It should be understood that the above only illustrates and describes examples whereby the present invention may be carried out, and that modifications and/or alterations may be made thereto without departing from the scope of the invention.

## Claims

1. A dew point sensor system for a humidifier (50) with a housing (52) comprising:
A) a controller (22) comprising a dew point calculator, wherein the dew point calculator is a look-up table for calculating a real-time dew point temperature and a target heater plate temperature;
B) an ambient temperature sensor (20) which generates ambient temperature data, the ambient temperature sensor (20) operatively-connected to the controller (22);
C) an air pathway (52) comprising;
i) a blower (38) within the air pathway (52), wherein the blower (38) causes air to move in the air pathway (52);
ii) an air flow sensor (26) within the air pathway (52), wherein the air flow sensor (26) generates air flow data about the air moving in the air pathway (52);
iii) a liquid reservoir (56) operatively-connected to the air pathway (52); and
iv) a component selected being an air pathway temperature sensor (28);
D) a heater plate (32) operatively-connected to heat the liquid reservoir (56), wherein the heater plate (32) comprises a heater plate temperature sensor (34), and wherein the heater plate temperature sensor (34) generates heater plate temperature data; and
E) an external wire (60) which is outside of the housing of the humidifier operatively-connecting the controller (22) and the component, wherein the ambient temperature sensor (20) is located on the external wire (60), wherein the heater plate (32), and the heater plate temperature sensor (34) are operatively-connected to the controller (22), and the blower (38) is operatively-connected to the controller (22), wherein the controller (22) is configured to receive the ambient temperature data, the air flow data, acquire a set dew point temperature , and calculate the real-time dew point temperature and the target heater plate temperature by employing the look-up table, wherein the real-time dew point temperature is calculated from the heater plate temperature data, the air flow data and the ambient temperature data, and the target heater plate temperature is calculated from the ambient temperature data, the air flow data, and the set dew point temperature.

2. The dew point sensor system according to claim 1, wherein the air pathway temperature sensor (28) is located in the air pathway (52) outlet downstream of the liquid reservoir (56); or wherein the air pathway temperature sensor (28) is located immediately downstream of the liquid reservoir (56); or wherein the air pathway temperature sensor (28) is located downstream of the liquid reservoir (56).

3. The dew point sensor system according to any one of the previous claims, further comprising an air pathway (52) humidity sensor located within the air pathway (52).

4. The dew point sensor system according to any one of the previous claims, wherein the component is the air pathway temperature sensor (28).

5. The dew point sensor system according to any one of the previous claims, wherein the heater plate temperature sensor (34) is directly-affixed to the heater plate (32).

6. The dew point sensor system according to any one of the previous claims, wherein the liquid reservoir (56) comprises a liquid; or water; or distilled water; or sterile water.

7. The dew point sensor system according to any one of the previous claims, further comprising a housing, wherein the air pathway (52), the heater plate (32) and the controller (22) are accommodated in the housing.

8. A method for modulating a humidifier heater plate (32) comprising the steps of:
A) providing a humidifier comprising the dew point sensor system according to any one of the previous claims;
B) generating ambient temperature data by the ambient temperature sensor (20);
C) transmitting the ambient temperature data by the ambient temperature sensor (20) to the controller (22);
D) generating air flow data by the air flow sensor (26);
E) transmitting the air flow data by the air flow sensor (26) to the controller (22);
F) generating heater plate temperature data by the heater plate temperature sensor (34);
G) transmitting the heater plate temperature data by the heater plate temperature sensor (34) to the controller (22);
H) acquiring the set dew point temperature for the controller (22);
I) receiving the ambient temperature data, the air flow data, and the heater plate temperature data by the controller (22);
J) calculating the real-time dew point temperature by the controller (22) by employing the look-up table, wherein the real-time dew point temperature is calculated from the ambient temperature data, the air flow data, and the heater plate temperature data;
K) calculating a target heater plate temperature by the controller (22) by employing the look-up table, wherein the target heater plate temperature is calculated from the ambient temperature data, the air flow data, and the set dew point temperature;
L) comparing the target heater plate temperature with the heater plate temperature data by the controller (22); and
M) adjusting the heater plate temperature by the controller (22) to the target heater plate temperature based on the target heater plate temperature and the heater plate temperature data, wherein the heater plate temperature is adjusted by the controller (22) adjusting a power flow to the heater plate (32).

9. The method according to claim 8, further comprising the step of activating the heater plate (32), and wherein the generating of heater plate temperature data in step (F) occurs after the heater plate (32) is activated.

10. The method according to Claim 8, wherein if the controller (22) discovers that the difference between the set dew point temperature data and the real-time dew point temperature is about ≥ 1 °C; or about ≥ 2 °C; or about ≥ 4 °C the heater plate (32) is activated and warmed up, and the controller (22) sounds an alarm;
wherein if the controller (22) discovers that the difference between the set dew point temperature data and the real-time dew point temperature is about ≥ 1 °C; or about ≥ 2 °C; or about ≥ 4 °C after the heater plate (32) is activated and warmed up for a continuous period of time of about ≥ 30 seconds; or about ≥ 1 minute; or about ≥ 2 minutes, and the controller (22) sounds an alarm;
wherein the adjusting of the heater plate temperature in step (M) takes place if the difference between the heater plate temperature and the target heater plate temperature is about ≥ 0.1 °C; or about ≥ 2 °C; or about ≥ 4 °C;
and wherein when the heater plate temperature is higher than the target plate temperature, the heater plate temperature is adjusted by the controller (22) decreasing the power flow to the heater plate (32), and when the heater plate temperature is lower than the target heater plate temperature, the heater plate temperature is adjusted by the controller (22) increasing the power flowing to the heater plate (32).

11. The method according to any one of Claims 8 to 10, comprising the step of repeating steps (B) to (M); or steps (B) to (M) are repeated at least about 600 times/minute; or from about 1 times/minute to about 1000 times/minute; or from about 10 times/minute to about 800 times/minute.

12. A humidifier comprising the dew point sensor system according to any one of Claims 1 to 7.

13. A medical device comprising: at least one of a continuous positive air pressure machine and an automatic positive air pressure machine; and a humidifier comprising the dew point sensor system according to any one of Claims 1 to 7.

## Patentansprüche

1. Taupunktsensorsystem für einen Befeuchter (50) mit einem Gehäuse (52), mit:
A) einem Regelgerät (22), das einen Taupunktrechner umfasst, wobei der Taupunktrechner eine Nachschlagetabelle zur Berechnung einer Echtzeit-Taupunkttemperatur und einer Soll-Heizplattentemperatur ist,
B) einem Umgebungstemperatursensor (20), der Umgebungstemperaturdaten erzeugt, wobei der Umgebungstemperatursensor (20) wirkungsmäßig mit dem Regelgerät (22) verbunden ist,
C) einem Luftpfad (52), der Folgendes umfasst:
i) ein Gebläse (38) in dem Luftpfad (52), wobei das Gebläse (38) bewirkt, dass sich Luft in dem Luftpfad (52) bewegt,
ii) einen Luftströmungssensor (26) in dem Luftpfad (52), wobei der Luftströmungssensor (26) Luftströmungsdaten über die sich in dem Luftpfad (52) bewegende Luft erzeugt,
iii) einen Flüssigkeitsbehälter (56), der mit dem Luftpfad (52) wirkungsmäßig verbunden ist, und
iv) eine ausgewählte Komponente, die ein Luftpfadtemperatursensor (28) ist,
D) einer Heizplatte (32), die wirkungsmäßig so verbunden ist, dass sie den Flüssigkeitsbehälter (56) erwärmt, wobei die Heizplatte (32) einen Heizplattentemperatursensor (34) umfasst und wobei der Heizplattentemperatursensor (34) Heizplattentemperaturdaten erzeugt, und
E) einem außenliegenden Kabel (60), das sich außerhalb des Gehäuses des Befeuchters befindet und das Regelgerät (22) und die Komponente wirkungsmäßig verbindet, wobei sich der Umgebungstemperatursensor (20) an dem außenliegenden Kabel (60) befindet, wobei die Heizplatte (32) und der Heizplattentemperatursensor (34) wirkungsmäßig mit dem Regelgerät (22) verbunden sind und das Gebläse (38) wirkungsmäßig mit dem Regelgerät (22) verbunden ist, wobei das Regelgerät (22) so eingerichtet ist, dass es die Umgebungstemperaturdaten, die Luftströmungsdaten empfängt, eine Einstell-Taupunkttemperatur erfasst und die Echtzeit-Taupunkttemperatur und die Soll-Heizplattentemperatur berechnet, indem es die Nachschlagetabelle verwendet, wobei die Echtzeit-Taupunkttemperatur aus den Heizplattentemperaturdaten, den Luftströmungsdaten und den Umgebungstemperaturdaten berechnet wird und die Soll-Heizplattentemperatur aus den Umgebungstemperaturdaten, den Luftströmungsdaten und der Einstell-Taupunkttemperatur berechnet wird.

2. Taupunktsensorsystem nach Anspruch 1, bei dem der Luftpfadtemperatursensor (28) im Auslass des Luftpfades (52) stromabwärts des Flüssigkeitsbehälters (56) gelegen ist oder bei dem der Luftpfadtemperatursensor (28) unmittelbar stromabwärts des Flüssigkeitsbehälters (56) gelegen ist oder bei dem der Luftpfadtemperatursensor (28) stromabwärts des Flüssigkeitsbehälters (56) gelegen ist.

3. Taupunktsensorsystem nach einem der vorhergehenden Ansprüche, ferner mit einem im Luftpfad (52) befindlichen Feuchtigkeitssensor des Luftpfads (52).

4. Taupunktsensorsystem nach einem der vorhergehenden Ansprüche, bei dem die Komponente der Luftpfadtemperatursensor (28) ist.

5. Taupunktsensorsystem nach einem der vorhergehenden Ansprüche, bei dem der Heizplattentemperatursensor (34) unmittelbar an der Heizplatte (32) befestigt ist.

6. Taupunktsensorsystem nach einem der vorhergehenden Ansprüche, bei dem der Flüssigkeitsbehälter (56) eine Flüssigkeit oder Wasser oder destilliertes Wasser oder steriles Wasser umfasst.

7. Taupunktsensorsystem nach einem der vorhergehenden Ansprüche, ferner mit einem Gehäuse, wobei der Luftpfad (52), die Heizplatte (32) und das Regelgerät (22) im Gehäuse untergebracht sind.

8. Verfahren zur Modulierung einer Befeuchterheizplatte (32), das die folgenden Schritte umfasst:
A) Bereitstellen eines Befeuchters, der das Taupunktsensorsystem nach einem der vorhergehenden Ansprüche umfasst,
B) Erzeugen von Umgebungstemperaturdaten durch den Umgebungstemperatursensor (20),
C) Übertragen der Umgebungstemperaturdaten durch den Umgebungstemperatursensor (20) an das Regelgerät (22),
D) Erzeugen von Luftströmungsdaten durch den Luftströmungssensor (26),
E) Übertragen der Luftströmungsdaten durch den Luftströmungssensor (26) an das Regelgerät (22),
F) Erzeugen von Heizplattentemperaturdaten durch den Heizplattentemperatursensor (34),
G) Übertragen der Heizplattentemperaturdaten durch den Heizplattentemperatursensor (34) an das Regelgerät (22),
H) Erfassen der Einstell-Taupunkttemperatur für das Regelgerät (22),
I) Empfangen der Umgebungstemperaturdaten, der Luftströmungsdaten und der Heizplattentemperaturdaten durch das Regelgerät (22),
J) Berechnen der Echtzeit-Taupunkttemperatur durch das Regelgerät (22), indem es die Nachschlagetabelle verwendet, wobei die Echtzeit-Taupunkttemperatur aus den Umgebungstemperaturdaten, den Luftströmungsdaten und den Heizplattentemperaturdaten berechnet wird,
K) Berechnen einer Soll-Heizplattentemperatur durch das Regelgerät (22), indem es die Nachschlagetabelle verwendet, wobei die Soll-Heizplattentemperatur aus den Umgebungstemperaturdaten, den Luftströmungsdaten und der Einstell-Taupunkttemperatur berechnet wird,
L) Vergleichen der Soll-Heizplattentemperatur mit den Heizplattentemperaturdaten durch das Regelgerät (22) und
M) Regulieren der Heizplattentemperatur auf die Soll-Heizplattentemperatur durch das Regelgerät (22) auf der Grundlage der Soll-Heizplattentemperatur und der Heizplattentemperaturdaten, wobei die Heizplattentemperatur dadurch reguliert wird, dass das Regelgerät (22) einen Leistungsfluss zu der Heizplatte (32) reguliert.

9. **Verfahren** nach Anspruch 8, ferner mit dem Schritt, bei dem die Heizplatte (32) aktiviert wird, und wobei das Erzeugen von Heizplattentemperaturdaten in Schritt (F) erfolgt, nachdem die Heizplatte (32) aktiviert ist.

10. Verfahren nach Anspruch 8, bei dem die Heizplatte (32), wenn das Regelgerät (22) feststellt, dass die Differenz zwischen den Einstell-Taupunkttemperaturdaten und der Echtzeit-Taupunkttemperatur etwa ≥ 1°C oder etwa ≥ 2°C oder etwa ≥ 4°C beträgt, aktiviert und aufgewärmt wird, und das Regelgerät (22) einen Alarm ertönen lässt,
wobei, wenn das Regelgerät (22) feststellt, dass die Differenz zwischen den Einstell-Taupunkttemperaturdaten und der Echtzeit-Taupunkttemperatur etwa ≥ 1°C oder etwa ≥ 2°C oder etwa ≥ 4°C beträgt, nachdem die Heizplatte (32) aktiviert und über einen durchgehenden Zeitraum von etwa ≥ 30 Sekunden oder etwa ≥ 1 Minute oder etwa ≥ 2 Minuten aufgewärmt wird, und das Regelgerät (22) einen Alarm ertönen lässt,
wobei das Regulieren der Heizplattentemperatur in Schritt (M) erfolgt, wenn die Differenz zwischen der Heizplattentemperatur und der Soll-Heizplattentemperatur etwa ≥ 0,1°C oder etwa ≥ 2°C oder etwa ≥ 4°C beträgt,
und wobei dann, wenn die Heizplattentemperatur höher als die Soll-Plattentemperatur ist, die Heizplattentemperatur dadurch reguliert wird, dass das Regelgerät (22) den Leistungsfluss zu der Heizplatte (32) verringert, und dann, wenn die Heizplattentemperatur niedriger als die Soll-Heizplattentemperatur ist, die Heizplattentemperatur dadurch reguliert wird, dass das Regelgerät (22) den Leistungsfluss zu der Heizplatte (32) erhöht.

11. Verfahren nach einem der Ansprüche 8 bis 10, mit dem Schritt, bei dem die Schritte (B) bis (M) wiederholt werden bzw. die Schritte (B) bis (M) wenigstens etwa 600 Mal/Minute oder von etwa 1 Mal/Minute bis etwa 1000 Mal/Minute oder von etwa 10 Mal/Minute bis etwa 800 Mal/Minute wiederholt werden.

12. Befeuchter mit dem Taupunktsensorsystem nach einem der Ansprüche 1 bis 7.

13. Medizinische Vorrichtung mit: einer Maschine für kontinuierlichen positiven Luftdruck und/oder einer Maschine für automatischen positiven Luftdruck und einem Befeuchter, der das Taupunktsensorsystem nach einem der Ansprüche 1 bis 7 umfasst.

## Revendications

1. Système capteur de point de rosée pour un humidificateur (50) présentant un boîtier (52), comprenant :
A) un contrôleur (22) comprenant un calculateur de point de rosée, le calculateur de point de rosée étant une table de correspondance pour calculer une température de point de rosée en temps réel et une température cible de la plaque chauffante ;
B) un capteur de température ambiante (20) qui génère des données de température ambiante, le capteur de température ambiante (20) étant relié de manière opérationnelle au contrôleur (22) ;
C) une voie d'air (52) comprenant :
i) une soufflante (38) à l'intérieur de la voie d'air (52), la soufflante (38) provoquant le déplacement de l'air dans la voie d'air (52) ;
ii) un capteur de débit d'air (26) à l'intérieur de la voie d'air (52), le capteur de débit d'air (26) générant des données de débit d'air sur l'air se déplaçant dans la voie d'air (52) ;
iii) un réservoir de liquide (56) relié de manière opérationnelle à la voie d'air (52) ; et
iv) un composant choisi comme étant un capteur de température de voie d'air (28) ;
D) une plaque chauffante (32) reliée de manière opérationnelle pour chauffer le réservoir de liquide (56), la plaque chauffante (32) comprenant un capteur de température de plaque chauffante (34), et le capteur de température de plaque chauffante (34) générant des données de température de plaque chauffante ; et
E) un fil externe (60) qui se trouve à l'extérieur du boîtier de l'humidificateur et reliant de manière opérationnelle le contrôleur (22) et le composant, le capteur de température ambiante (20) se trouvant sur le fil externe (60), la plaque chauffante (32) et le capteur de température de plaque chauffante (34) étant reliés de manière opérationnelle au contrôleur (22), et la soufflante (38) étant reliée de manière opérationnelle au contrôleur (22), le contrôleur (22) étant réalisé de manière à recevoir les données de température ambiante, les données de débit d'air, à acquérir une température de consigne de point de rosée, et à calculer la température de point de rosée en temps réel et la température cible de la plaque chauffante en utilisant la table de correspondance, la température de point de rosée en temps réel étant calculée à partir des données de température de plaque chauffante, des données de débit d'air et des données de température ambiante, et la température cible de la plaque chauffante étant calculée à partir des donnés de température ambiante, des données de débit d'air et de la température de consigne de point de rosée.

2. Système capteur de point de rosée selon la revendication 1, dans lequel le capteur de température de voie d'air (28) est agencé dans la sortie de la voie d'air (52), en aval du réservoir de liquide (56) ; ou dans lequel le capteur de température de voie d'air (28) est agencé immédiatement en aval du réservoir de liquide (56) ; ou dans lequel le capteur de température de voie d'air (28) est agencé en aval du réservoir de liquide (56).

3. Système capteur de point de rosée selon l'une des revendications précédentes, comprenant en outre un capteur d'humidité de la voie d'air (52) agencé à l'intérieur de la voie d'air (52).

4. Système capteur de point de rosée selon l'une des revendications précédentes, dans lequel le composant est le capteur de température de voie d'air (28).

5. Système capteur de point de rosée selon l'une des revendications précédentes, dans lequel le capteur de température de plaque chauffante (34) est directement fixé à la plaque chauffante (32).

6. Système capteur de point de rosée selon l'une des revendications précédentes, dans lequel le réservoir de liquide (56) comprend un liquide, ou de l'eau, ou de l'eau distillée, ou de l'eau stérile.

7. Système capteur de point de rosée selon l'une des revendications précédentes, comprenant en outre un boîtier, la voie d'air (52), la plaque chauffante (32) et le contrôleur (22) étant logés dans le boîtier.

8. Procédé de modulation d'une plaque chauffante d'humidificateur (32), comprenant les étapes suivantes :
A) fournir un humidificateur comprenant le système capteur de point de rosée selon l'une des revendications précédentes ;
B) générer des données de température ambiante au moyen du capteur de température ambiante (20) ;
C) transmettre les données de température ambiante du capteur de température ambiante (20) au contrôleur (22) ;
D) générer des données de débit d'air au moyen du capteur de débit d'air (26) ;
E) transmettre les données de débit d'air du capteur de débit d'air (26) au contrôleur (22) ;
F) générer des données de température de plaque chauffante au moyen du capteur de température de plaque chauffante (34) ;
G) transmettre les données de température de plaque chauffante du capteur de température de plaque chauffante (34) au contrôleur (22) ;
H) acquérir la température de consigne de point de rosée pour le contrôleur (22) ;
I) recevoir les données de température ambiante, les données de débit d'air et les données de température de plaque chauffante du contrôleur (22) ;
J) calculer la température de point de rosée en temps réel au moyen du contrôleur (22) en utilisant la table de correspondance, la température de point de rosée en temps réel étant calculée à partir des données de température ambiante, des données de débit d'air et des données de température de plaque chauffante ;
K) calculer une température cible de plaque chauffante au moyen du contrôleur (22) en utilisant la table de correspondance, la température cible de plaque chauffante étant calculée à partir des données de température ambiante, des données de débit d'air et de la température de consigne de point de rosée ;
L) comparer la température cible de plaque chauffante avec les données de température de plaque chauffante au moyen du contrôleur (22) ; et
M) régler la température de plaque chauffante au moyen du contrôleur (22) à la température cible de plaque chauffante en fonction de la température cible de plaque chauffante et des données de température de plaque chauffante, la température de la plaque chauffante étant réglée en ce que le contrôleur (22) ajuste un flux d'énergie vers la plaque chauffante (32).

9. Procédé selon la revendication 8, comprenant en outre l'étape d'activation de la plaque chauffante (32), et dans lequel la génération des données de température de plaque chauffante à l'étape (F) est réalisée après l'activation de la plaque chauffante (32).

10. Procédé selon la revendication 8, dans lequel si le contrôleur (22) découvre que la différence entre les données de température de consigne de point de rosée et la température de point de rosée en temps réel est environ ≥ 1°C ; ou environ ≥ 2°C ; ou environ ≥ 4°C, la plaque chauffante (32) est activée et chauffée, et le contrôleur (22) émet une alarme ;
si le contrôleur (22) découvre que la différence entre les données de température de consigne de point de rosée et la température de point de rosée en temps réel est environ ≥ 1°C ; ou environ ≥ 2°C ; ou environ ≥ 4°C après l'activation et le chauffage de la plaque chauffante (32) pendant une période continue d'environ ≥ 30 secondes ; ou d'environ ≥ 1 minute ; ou d'environ ≥ 2 minutes, le contrôleur (22) émet une alarme ;
le réglage de la température de plaque chauffante à l'étape (M) étant réalisé si la différence entre la température de plaque chauffante et la température cible de plaque chauffante est environ ≥ 1°C ; ou environ ≥ 2°C ; ou environ ≥ 4°C ;
et si la température de plaque chauffante est supérieure à la température cible de plaque chauffante, la température de plaque chauffante est réglée en ce que le contrôleur (22) diminue le flux d'énergie vers la plaque chauffante (32), et si la température de plaque chauffante est inférieure à la température cible de plaque chauffante, la température de plaque chauffante est réglée en ce que le contrôleur (22) augmente le flux d'énergie vers la plaque chauffante (32).

11. Procédé selon l'une des revendications 8 à 10, comprenant l'étape de répétition des étapes (B) à (M) ; ou les étapes (B) à (M) étant répétées au moins environ 600 fois/minute ; ou d'environ 1 fois/minute à environ 1000 fois/minute ; ou d'environ 10 fois/minute à environ 800 fois/minute.

12. Humidificateur comprenant le système capteur de point de rosée selon l'une des revendications 1 à 7.

13. Dispositif médical comprenant : au moins un appareil à pression d'air positive continue et un appareil à pression d'air positive automatique ; et un humidificateur comprenant le système capteur de point de rosée selon l'une des revendications 1 à 7.
